(19) **Europäisches Patentamt**

**European Patent Office**

**Office européen des brevets**

(11) **EP 0 913 406 B1**

(12) **EUROPEAN PATENT SPECIFICATION**

(45) Date of publication and mention
of the grant of the patent:
**07.07.2004 Bulletin 2004/28**

(51) Int Cl.⁷: **C08B 31/04**, A61K 9/20,
A23L 1/035

(21) Application number: **98120607.1**

(22) Date of filing: **30.10.1998**

(54) **Glucoamylase converted starch derivatives and their use as emulsifying and encapsulating agents**

Mit Glukoamylase umgesetzte Stärkederivate und ihre Verwendung als Emulgatoren und Einkapselungsmittel

Dérivés d'amidon convertis par la glucoamylase et l'application comme agents émulsifiants ou comme agents d'encapsulation

(84) Designated Contracting States:
**AT BE CH DE DK ES FI FR GB GR IE IT LI LU MC NL PT SE**

(30) Priority: **31.10.1997 US 962285**
**23.10.1998 US 178087**

(43) Date of publication of application:
**06.05.1999 Bulletin 1999/18**

(73) Proprietor: **National Starch and Chemical Investment Holding Corporation**
**Wilmington, Delaware 19803-7663 (US)**

(72) Inventors:
 • **Blue, Emily Keller**
  **Indianapolis, Indiana 46268 (US)**
 • **Chiu, Chung-Wai**
  **Westfield, New Jersey 07090 (US)**
 • **Hussain, Zahera**
  **Hyde Park, New York 12538 (US)**
 • **Shah, Himanshu**
  **Branchburg, New Jersey 08876 (US)**
 • **Trubiano, Paul**
  **Somerville, New Jersey 08876 (US)**
 • **Boyd, Dennis**
  **Neshanic Station, New Jersey 08853 (US)**

(74) Representative:
**Held, Stephan, Dr.rer.nat., Dipl.-Chem. et al**
**Meissner, Bolte & Partner**
**Postfach 86 03 29**
**81630 München (DE)**

(56) References cited:
 US-A- 3 455 838    US-A- 4 035 235
 US-A- 4 845 035    US-A- 5 194 284
 US-A- 5 445 950    US-A- 5 468 286

 • PATENT ABSTRACTS OF JAPAN vol. 97, no. 2, 28 February 1997 & JP 08 283303 A (CHIBA SEIFUN) & DATABASE WPI Week 9702 Derwent Publications Ltd., London, GB; AN 017385

**EP 0 913 406 B1**

**Description**

The present invention relates to a modified starch which is prepared by hydrolysis of a starch molecule using glucoamylase after the preparation of a starch derivative containing a hydrophobic group or both a hydrophobic and a hydrophilic group. Such modified starch is useful as an emulsifying agent or as an encapsulating agent, particularly in systems where high load and retention of the active ingredient, low surface oil exposure, and excellent oxidation resistance is desired. The encapsulating agent is useful in numerous applications, including a tablet.

US-A-4,977,252 and US-A-5,185,176 disclose starch derivatives containing a hydrophobic or both a hydrophobic and a hydrophilic group which have been enzymatically degraded by exo-enzymes which exhibit selectivity in cleaving the 1,4-linkages and leaving the 1,6-linkages intact. These modified starches are useful as emulsifiers.

A variety of chemical compositions are conventionally used as encapsulating agents in, *inter alia*, the food, cosmetic, paint, pharmaceutical, personal care, and polymer industries. Typical compositions which conventionally function as encapsulating agents include gum arabic, dextrins, low viscosity modified starches, arabinogalactan, gum acacia, casein, gelatin, carboxymethyl cellulose, and tragacanth, karaya, sodium alginate, tannin, and celluloses.

These typical compositions however do not consistently provide high active agent loading and retention, low surface oil and excellent oxidation resistance. In general, powders prepared with conventional encapsulating agents do not contain a high level of active agents. When loaded with oil levels of higher than 15-20%, such conventional encapsulated powders lose a considerable amount of the oil during the drying process, have much of the oil exposed on the surface of the powder, and/or generally have poor oxidation resistance.

U.S.-A-3,971,852 discloses a method for encapsulating oils in particles of a solid water-sensitive, preferably water-soluble, protective matrix that isolates the oils until they are released for use by exposure of the particles to moisture. The matrix-forming encapsulation materials include mixtures of polysaccharides and polyhydroxy compounds that can form aqueous emulsions with the oil. Although the patent claims efficient encapsulation of up to 80% by volume and surface oil not substantially above 5%, with a relatively high loading, the known process fails to provide efficient encapsulation oil recovery with excessive oil loss during drying and extractable oils as high as 10-24% when the encapsulated oil content exceeds 60% by weight. Furthermore, it has not been shown that these matrices provide good oxidation resistance.

US-A-5,087,461 discloses a spray dried composition encapsulated in an extruded glassy matrix composed of a chemically modified starch having a dextrose equivalent no greater than 2, a maltodextrin, a corn syrup solid or polydextrose, and a mono- or di-saccharide. However, these encapsulated products are unable to achieve high loading and are susceptible to oxidation.

EP-A-550 067 discloses a method for encapsulating oils in a water-sensitive cellular solid matrix by drying an aqueous emulsion containing the oil to be encapsulated, a non-crosslinked lipophilically modified starch that undergoes crosslinking during drying, and a polyhydroxy compound that forms with the polysaccharide material a continuous aqueous phase in which the oil is dispersible as a discontinuous phase. This method of encapsulation is unacceptable for foods and pharmaceuticals and requires the incorporation of a silicone-based material which is difficult to process using conventional methods. Furthermore, it has not been shown that these matrices provide good oxidation resistance.

US-A-3,455,838 ist directed to an encapsulating agent consisting essentially of the dextrinized starch acid-ester of a substituted dicarboxylic acid. That document is directed to a dextrin.

JP-A-8283303 describes an emulsifier comprising a higher fatty acid dextrin formed by introducing a $C_{10-24}$ saturated fatty acid into dextrin having an average degree of saccharide polymerization of 5-150. That document is also directed to dextrins.

However, none of the encapsulating agents known in the art provide a high load of at least 40% while maintaining a retention of at least 90% of the active ingredient with less than 3% surface oil exposure, and excellent oxidation resistance for a wide variety of encapsulating agents.

Compressed tablets are also well-known, particularly in the pharmaceutical industry. Known methods of tabletting include direct compression and wet or dry granulation followed by compression. Tablet formulations characteristically should be free flowing, cohesive and lubricating. Sometimes, it is desired to encapsulate a component of the tablet and gelatin is considered as a standard encapsulating agent in many industries.

However, consumers may desire products which do not contain gelatin for a variety of reasons including dietary to meet strict Kosher, Halal or vegetarian standards. Many consumers also want to avoid bovine products because of the current scare over Bovine Spongiform Encephalopathy (Mad Cow Disease). Further, gelatin is an expensive excipient and its replacement is desirable to reduce the cost of the product.

It is known in the art that certain starches are excellent encapsulating agents. However, as starches are generally used as disintegrants, impeding compression and hardness of the tablet, starch encapsulating agents are not generally used in significant quantities in tablets. Hardness is necessary in a tablet as it provides resistance to chipping, abrasion, and breakage under conditions of storage, transportation, and handling prior to consumer con-

2

sumption.

Other encapsulating agents generally do not allow for good compressibility while providing high load and retention of the active agent and oxidative resistance.

Surprisingly, it has now been discovered that the present invention which uses a modified starch, prepared by enzymatically converting a starch using glucoamylase after the preparation of a starch derivative containing a hydrophobic group or a hydrophobic and a hydrophilic group, as an encapsulating agent, may consistently allow for such high load and retention of a variety of active ingredients and low oil exposure while providing excellent oxidation resistance. Further, such glucoamylase degraded starch derivatives are excellent emulsifiers. It has also been discovered that the encapsulating agent comprising such modified starch allows similar compressibility characteristics and resultant hardness to gelatin in a tablet formulation.

The present invention is directed to a modified starch which is prepared by enzymatic hydrolysis of a starch molecule using glucoamylase after the preparation of a starch derivative containing a hydrophobic group or both a hydrophobic and a hydrophilic group. Such modified starch is useful as an emulsifying agent and as an encapsulating agent, particularly in systems where high load and retention of the active ingredient, low surface oil exposure, and excellent oxidation resistance is desired.

An object of the present invention is to provide a glucoamylase converted, starch derivative containing a hydrophobic group or a hydrophobic and a hydrophilic group and a method of producing such starch.

Another object of the present invention is to provide a glucoamylase hydrolyzed, hydrophobically derivatized starch.

Still another object of the present invention is to provide a glucoamylase hydrolyzed, alkenyl succinic starch.

Yet another object of the present invention is to provide a glucoamylase converted, starch derivative containing a hydrophobic group or a hydrophobic and a hydrophilic and a method of producing such starch.

Still yet another object of the present invention is to provide products containing a glucoamylase converted, starch derivative containing a hydrophobic group or a hydrophobic and a hydrophilic group as an emulsifier or as an encapsulating agent and a method of preparing such products.

A further object of the present invention is to provide products containing a glucoamylase hydrolyzed, hydrophobically derivatized starch as an emulsifying or as an encapsulating agent.

A still further object of the present invention is to provide products containing a or glucoamylase hydrolyzed, alkenyl succinic starch as an emulsifying or as an encapsulating agent.

An additional object is to provide a tablet, such as a pharmaceutical tablet, which contains such encapsulating agents.

These and other objects of the present invention will become apparent to one skilled in the art from the following detailed description and examples below.

The present invention is directed to a modified starch which is prepared by enzymatic hydrolysis of a starch molecule using glucoamylase after the preparation of a starch derivative containing a hydrophobic group or both a hydrophobic and a hydrophilic group. Such modified starch is useful as an emulsifying and/or as an encapsulating agent. In particular, these starches are useful as encapsulating agents in systems where high load and retention of the active ingredient, low surface oil exposure, and excellent oxidation resistance is desired. Further, such encapsulating agents can be processed at high solids during the encapsulation process.

The present invention is also directed to a tablet, particularly a pharmaceutical dosage form, which contains the encapsulating agent. The encapsulating agent allows for good compressibility and hardness of the tablet. Further, it may allow for high load and retention of a variety of active agents as well as oxidative resistance.

All starches and flours are suitable for use herein and may be derived from any native source. A native starch or flour, as used herein, is one as it is found in nature, including those developed by plant breeding, and bioengineered starches. Typical sources for the starches and flours are cereals, tubers, roots, legumes and fruits. The native source can be corn, pea, potato, sweet potato, banana, barley, wheat, rice, sago, amaranth, tapioca, arrowroot, canna, sorghum, and waxy or high amylose varieties thereof. As used herein, the term "waxy" is intended to include a starch or flour containing at least about 95% by weight amylopectin and the term "high amylose" is intended to include a starch or flour containing at least about 45% by weight amylose. In particular, corn, waxy maize, tapioca, potato, and rice are useful in the instant invention.

Also included as useful base starch materials are the conversion products derived from any of the above starches including fluidity or thin-boiling starches prepared by oxidation, α-amylase conversion, mild acid hydrolysis or heat dextrinization, and derivatized starch such as ethers and esters.

A particularly useful starch base is a gelatinized starch, that is a precooked, non-granular starch, and also may be a fluidity starch converted by mild acid degradation or heat dextrinization methods that are well known in the art. For example, see Rutenberg, "Starch and Its Modifications," *Handbook of Water-Soluble Gums and Resins,* Davidson, Editor, McGraw-Hill, Inc., New York, N.Y., 1980, pp.22-36. A combination of one or more of these conversion techniques may be used. The conversion is typically carried out before treatment with a hydrophobic or a hydrophobic/hydrophilic reagent and before the enzyme treatment. If desired, the starch base may be converted by treatment with an α-amylase enzyme to produce a fluidity starch in the manner disclosed in US-A-4,035,235. Where a high viscosity system is desired, it is not necessary to convert the base starch.

The starch may be derivatized by treatment with any reagent or combination of reagents which contributes emulsifying and/or encapsulating properties to the starch. The reagent must contain a hydrophobic moiety and may contain a hydrophilic moiety. The hydrophobic moiety should be an alkyl or an alkenyl group which contains at least five carbon atoms or an aralkyl or aralkenyl group which contains at least six carbon atoms, particularly up to about twenty-four carbon atoms. The hydrophilic moiety may be contributed by the reagent or the starch's own hydroxyl groups may serve as the hydrophilic moiety and the reagent may contribute only the hydrophobic moiety.

Any process for derivatizing starch which yields the desired blend of hydrophobic or hydrophobic and hydrophilic functions on the starch molecule and thereby yields stable encapsulation properties may be used to prepare the modified starch of the present invention. Suitable derivatives and methods for producing them are known in the art and disclosed in US-A-4,626,288. In a particularly useful embodiment, the starch is derivatized by reaction with an alkenyl cyclic dicarboxylic acid anhydride by the method disclosed in US-A-2,613,206 and US-A-2,661,349, or propylene oxide, more particularly by reaction with octenylsuccinic anhydride.

Where a low viscosity is desirable, a particularly useful embodiment is an octenyl succinic half ester derivative of an amylopectin containing starch, such as waxy maize, which has been converted to a water fluidity (WF) of up to 60. Water fluidity is an empirical test of viscosity measured on a scale of 0-90 wherein fluidity is the reciprocal of viscosity. Water fluidity of starches is typically measured using a Thomas Rotational Shear-type Viscometer (commercially available from Arthur A. Thomas CO., Philadelphia, PA), standardized at 30°C with a standard oil having a viscosity of 24.73 mPas (cps) which oil requires 23.12±0.05 sec for 100 revolutions. Accurate and reproducible measurements of water fluidity are obtained by determining the time which elapses for 100 revolutions at different solids levels depending on the starch's degree of conversion: as conversion increases, the viscosity decreases. In a particularly useful embodiment, the converted starch is treated with at from 0.1 % to 3.0% for food products and at least 0.1% for other products, of the octenyl succinic anhydride. In the alternative, a hydroxypropyl octenyl succinic derivative may be used.

For other products, any degree of substitution or level of conversion that results in the desired viscosity and encapsulation properties may be employed. For example, US-A-4,035,235 disclosed a suitable embodiment comprising a method for producing a hydrophobic derivative of starch to be used as an alternative to gum arabic in encapsulating water insoluble substances, such as volatile flavoring oils and perfumes.

After derivatizing the starch, it is further enzymatically hydrolyzed by glucoamylase. The enzymatic hydrolysis of the starch base is carried out using techniques known in the art. The amount of enzyme used is dependent upon the enzyme source and activity, base material used, and the amount of hydrolysis desired. Typically, the enzyme is used in an amount of from 0.01 to 1.0%, particularly from 0.01 to 0.3%, by weight of the starch.

The optimum parameters for enzyme activity will vary depending upon the enzyme used. The rate of enzyme degradation depends upon factors known in the art, including the enzyme concentration, substrate concentration, pH, temperature, the presence or absence of inhibitors, and the degree and type of modification. These parameters may be adjusted to optimize the digestion rate of the starch base.

The starch may be gelatinized before glucoamylase hydrolysis. The gelatinization process unfolds the starch molecules from the granular structure, thereby permitting the enzyme to more easily and uniformly degrade the starch molecules. However, as glucoamylase can hydrolyze granular starch, gelatinization is not necessary.

Generally the enzyme treatment is carried out in an aqueous or buffered slurry at a starch solids level of 10 to 40%, depending upon the base starch being treated. A solids level of from 15 to 35% is particularly useful, from 18 to 25% more particularly useful, in the instant invention. In the alternative, the process may utilize an enzyme immobilized on a solid support.

Typically, enzyme digestion is carried out at the highest solids content feasible without reducing reaction rates in order to facilitate any desired subsequent drying of the starch composition. Reaction rates may be reduced by high solids content as agitation becomes difficult or ineffective and the starch dispersion becomes more difficult to handle.

The pH and temperature of the slurry should be adjusted to provide effective enzyme hydrolysis. These parameters are dependent upon the enzyme to be used and are known in the art. In general, a temperature of 22 to 65°C is used, particularly from 50 to 62°C. In general, the pH is adjusted to 3.5 to 7.5, particularly from 4.0 to 6.0, using techniques known in the art.

The enzyme reaction is continued until a dextrose equivalent of at least 20 and up to 80, particularly 30 to 50, has been achieved, or until the desired end point (i.e., sufficient degradation to provide the desired functionality for the particular application) has been reached. The end point may be determined by a change in viscosity, by reducing sugar content (such as measured by dextrose equivalents), or by any other method known in the art for measuring the level of enzyme degradation of the starch molecule. In general, the enzyme reaction will take from 0.1 to 24 hours, particularly 0.5 to 4 hours. The time of the reaction is dependent upon the type of starch used, the amount of enzyme used, and the reaction parameters of solids percent, pH, and temperature.

The enzyme degradation is then terminated by any technique known in the art such as acid or base deactivation, heat deactivation, ion exchange, and solvent extraction. For example, acid deactivation may be accomplished by adjusting the pH to lower than 2.0 for at least 30 minutes or heat deactivation may be accomplished by raising the temperature to 85 to 95°C and maintaining it at that temperature for at least about 10 minutes to fully deactivate the enzyme. Heat deactivation is not suitable if a granular product is desired as the heat necessary to deactivate the enzyme will generally also gelatinize the starch.

The resultant solution is typically adjusted to the desired pH according to its intended end use. In general, the pH is adjusted to from 5.0 to 7.5, particularly from 6.0 to 7.0, using techniques known in the art. The modified starch is then typically dried using methods known in the art, particularly spray drying. However, the modified starch may also be used as a liquid concentrate.

The resulting starch is characterized by a relatively low viscosity, moderately high dextrose equivalent, neutral taste, and by its unique functionality as an encapsulating agent.

The viscosity of the resultant starch should be less than 30 seconds, particularly from 8 to 25 seconds, more particularly from 8 to 15 seconds as measured by the funnel method. Viscosity is an important parameter in contributing to efficient encapsulation.

To measure the viscosity of the starch is measured by the funnel method. The starch dispersion to be tested is adjusted to 19% or 25% (w/w) measured by refractometer. The temperature of the dispersion is controlled at 22°C. A total of 100 ml of the starch dispersion is measured into a graduated cylinder. It is then poured into a calibrated funnel while using a finger to close the orifice. A small amount is allowed to flow into the graduate to remove any trapped air and the balance is poured back into the funnel. The graduated cylinder is then inverted over the funnel so that the contents draw (flow) into the funnel while the sample is running. Using a timer, the time required for the 100 ml sample to flow through the apex of the funnel is recorded.

The glass portion of the funnel is a standard 58°, thick-wall, resistance glass funnel whose top diameter is 9 to 10 cm with the inside diameter of the stem being about 0.381 cm. The glass stem of the funnel is cut to an approximate length of 2.86 cm from the apex, carefully fire-polished, and refitted with a long stainless steel tip with is about 5.08 cm long with an outside diameter of about 0.9525 cm. The interior diameter of the steel tip is about 0.5952 cm at the upper end where is attached to the glass stem and about 0.4445 cm at the outflow end with the restriction in the width occurring at about 2.54 cm from the ends. The steel tip is attached to the glass funnel by means of a Teflon tube. The funnel is calibrated so as to allow 100 ml of water to go through in six seconds using the above procedure.

The resultant starch should have a dextrose equivalent of at least 20 and up to 80, particularly from 30 to 50. Dextrose equivalent (DE) is defined as the reducing power of the hydrolyzate. Each starch molecule has one reducing end: therefore DE is inversely related to molecular weight. The DE of anhydrous D-glucose is defined as 100 and the DE of unhydrolyzed starch is virtually zero.

The resultant starch should have a relatively high percent of sugars, at least 20 and up to 80% sugar, particularly from 30 to 40% glucose, more particularly from 30 to 35% glucose.

The active agent may be any substance which will not react with the starch system, including but not limited to oils, fats, flavors, colors, fragrances, vitamins, and pharmaceuticals. In particular, the modified starch of the present invention is useful for emulsifying or encapsulating oil-based active agents such as flavor oils and vitamins. These oils may be volatile or non-volatile and are generally characterized by being water immiscible but dispersible (emulsifiable) in water in the presence of an encapsulating agent.

The resultant starches, when used as emulsifying agents, have the advantages of improved shelf stability and resistance to oiling, gelling, and ringing during storage.

The resultant starches, when used as encapsulating agents, have the advantages of achieving and maintaining consistently high load levels, low oil exposure, and excellent oxidation resistance.

The active agents may be encapsulated using the modified starches of the present invention and techniques known in the art, including but not limited to spray drying, extrusion, spray chilling, and fluid bed coating. For example, the starch may be dispersed in water, the active agent may be added and emulsified, and the emulsion may then be spray dried to form the encapsulated product.

The encapsulated product prepared with the present encapsulating agents consistently achieve and maintain a relatively high load level of the active agent. The load level of the active agent realized may be greater than 40%, particularly greater than 50%, more particularly greater than 60%, by weight of the encapsulating agent. The level of active agent retained may be determined by methods known in the art such as by hydro-distillation in the case of flavor oils or by solvent extraction alone in the case of vitamins.

A high load level of active agent is desirable to reduce the cost of producing the final product as encapsulating agents are often expensive. Further, some encapsulating agents may contribute adverse or undesirable properties to the final system and it is thus desirable to reduce the amount of encapsulating agent used.

It is important not only to achieve a high load of active agent, but also to maintain it so as to enable a longer shelf life. Many active agents are volatile and/or labile, particularly flavors and fragrances. When the active agents are not encapsulated, they may be lost, producing undesirable variations in taste and aroma of the final products as perceived by the consumer. In addition, losses of such components increase the cost of the final products since it is necessary to increase the amount of the volatile/labile component to compensate for the losses which occur, and many are expensive.

In the case of oil as an active agent, the present encapsulating agents also retain the oil so as to provide a low surface oil. This is particularly true when glucoamylase is used to enzymatically hydrolyze the starch. The surface oil may be measured by methods known in the art such as by washing the encapsulated powder with a suitable solvent. Reduction of surface oil is important as increased surface oil indicates that the load of the active agent is not being maintained and inefficiency of encapsulation. Thus, reduction of surface oil results in a longer shelf life.

The present encapsulating agents also provide a relatively high level of oxidation resistance, thereby prolonging storage stability of the encapsulated product and shelf life of the final product. Oxidation resistance may be measured by methods known in the art. For example, oxidation resistance of encapsulating agents containing citrus oil may be determined by using gas chromatography (GC) to measure the amount of oxidization products of limonene, such as carvone, carviol, and limonene oxide, present in the oil extracted from powders aged at 50°C for two weeks: less than about 0.8% carvone typically indicates acceptable levels of oxidation. Oxidation resistance is important not only for flavor considerations of the oil, but also to maintain the activity of various vitamins. To further increase oxidation resistance, an anti-oxidant may be added to the oil.

The encapsulated product is effective when stored as a powder and spontaneously releases the active agent upon exposure to moisture. The resultant encapsulated product may be used at any level desired in food products, the amount being dependent upon the amount of active agent to be incorporated. In general, the starch will be used in an amount of from 0.01 to 10%, particularly from 0.1 to 5% by weight of the food product.

The resultant starch can be used in various food products including, but not limited to, cereals; powdered drink mixes; instant coffees and teas; powdered sauce and gravy mixes; instant soups; cereals; powdered dressings; bakery products; flavors; fragrances; colorants; and other dry food products. Upon preparation of these powdered and instant products, the moisture triggers the release mechanism, providing the active agent to the consumer.

The resultant starch may also be used in a variety of pharmaceuticals including vitamins; personal care products including antiperspirants, deodorants, soaps, fragrances, and cosmetics; hair care products, such as hair sprays, mousses, shampoos, cream rinses, and gels; paper products such as diapers, sanitary napkins, paper towels, tissues, toilet tissues; animal care products such as kitty litter; and household products such as carpet cleaners, and air fresheners.

The encapsulated product may also be used in a solid tablet-like form for a variety of applications, including detergents, foods and beverages, bath oils, agricultural products, and pharmaceuticals. The encapsulated products are particularly suitable for pharmaceutical tablets, including effervescent tablets.

The encapsulated product may generally be used at the desired level, the amount being dependent upon the amount of active agent to be incorporated, the desired hardness of the tablet, and the oxidative resistance desired. In general, the encapsulated product will be used in an amount of from 1 to 95% by weight of the tablet allowing for the active agent to be incorporated in an amount of from 1 to 60, particularly from 10 to 50%, by weight of the tablet.

The encapsulated product is particularly useful in a compressed tablet. The compressed tablet may be made using any method known in the art, particularly by direct compression of the tablet components. In the alternative, the tablet may be prepared by dry blending the encapsulated product with the other components of the formulation, granulating the mixture such as by fluid bed technology, roller compactor, extrusion, or high shear granulator, and dry compacting to a tablet.

Pharmaceutical excipients known in the art may be added to the pharmaceutical dosage form to impart satisfactory processing, compression, and disintegration characteristics to the formulation. Such excipients include, but are not limited to, diluents, flow enhancer, binders, lubricants and glidants, disintegrants, colors, flavors and sweetening agents. These excipients are well known in the art and are limited only by compatibility and characteristics desired.

Binders for the present invention include gelatin, microcrystalline cellulose, sugars, carboxymethyl cellulose, methyl cellulose, polyvinyl pyrrolidone, acacia, alginic acid, guar gum, hydroxypropyl methylcellulose, polyethylene oxide and ethyl cellulose.

Lubricants and glidants include talc, magnesium stearate, calcium stearate, stearic acid, glyceryl behenate, mineral oil, polyethylene glycol, sodium stearyl fumarate, stearic acid, vegetable oil, zinc stearate, and silicon dioxide.

Disintegrants suitable for the present invention include starches, algins, gums, croscarmelose, crospovidone, sodium starch glycolate, sodium laurel sulfate, microcrystalline cellulose, polacrilin potassium, and methylcellulose.

Diluents suitable for the present invention include dicalcium phosphate, calcium sulfate, lactose, cellulose, Kaolin, mannitol, sodium chloride, starch, sugars, calcium carbonate, calcium phosphate, dextrates, dextrin, dextrose, fructose, sorbitol, sucrose, and microcrystalline cellulose.

In particular, a binder is added to the tablet formulation to provide a tablet with the desired hardness. In general the hardness of the resultant tablet is at least 3, more particularly at least 4, most particularly at least 6 kilopascals (kP).

If the final desired product is other than a pharmaceutical dosage form, alternative additives known to those arts may be present. For example, flavors and fragrances in a bath oil tablet or surfactants in a detergent tablet.

Upon contact with water, the moisture triggers the release mechanism, allowing the active agent to be released from the encapsulating starch. For example, upon digestion of the pharmaceutical dosage forms, the active agent is released to the body.

The following examples are presented to further illustrate and explain the present invention and should not be taken as limiting in any regard. The following analytical tests were used to measure various parameters in the examples.

Determination of Dextrose Equivalents (DE)

The dextrose equivalent of starch may be determined by using the Reducing Sugars test described in Food Chemicals Codex, 4th ed., July 1, 1996. Section 5, General Tests and Assays, Appendix X: Carbohydrates (Starches, Sugars, and Related Substances) or Standard Analytical Method #E-26 for Dextrose Equivalent from the Corn Refiners Association.

Oxidation Resistance Analysis

Twenty (20) grams of the modified starch powder loosely filled, were placed in a one liter jar with a powder to air ratio of 1:25. The jar was capped tightly with Teflon. The sample was placed in a 50°C oven for two weeks.

The oil was then distilled using hydro-distillation. The distilled oil was then analyzed using gas chromatography for fresh (limonene) and oxidized (carvone) components.

Surface Oil Analysis

The unencapsulated oil present on the surface of the particle is repeatedly extracted using organic solvents, such as pentane, to remove all the surface oil and the extracted oil is quantitatively determined using gas chromatography techniques known in the art.

Oil Retention (Loading) Analysis

To determine the oil retention of the encapsulated product, 15 grams of the spray dried, encapsulated oil and 150 ml distilled water are mixed to reconstituted the emulsion. The emulsion is heated to reflux and held for four hours. The mixture is then cooled and the separated oil is removed and weighed.

$$\%\text{Retention} = \frac{\text{volume of oil extracted} \times \text{specific gravity of oil}}{\text{theoretical oil weight}} \times 100$$

Example 1 - Preparation of the Derivatized Starch

500 grams of waxy maize starch were slurried in 750 ml water. The pH was adjusted to 7.5 using 3% sodium hydroxide. 15 grams of octenylsuccinic anhydnde (OSA) were added in one-third increments every thirty minutes while maintaining the pH at 7.5 using 3% sodium hydroxide with constant agitation. The starch was then filtered out and washed with 750 ml water. The starch was then reslurried in 500 ml water and the pH adjusted to 5.5 with 3:1 hydrochloric acid. The starch was then filtered, washed with 750 ml water, and air dried to produce an OSA starch.

Example 2 - Preparation of the Modified Starch

100 grams of the OSA starch of Example 1 were slurried in 300 ml water and the pH adjusted to 5.5 using dilute hydrochloric acid. The slurry was gelatinized by jet cooking in a C1-339 jet cooker, commercially available from National Starch and Chemical Company, at 148.9°C (300°F), at a chamber pressure of 3.85 kg/cm$^2$ (55 psi) and a slurry rate of 6ml/min with the steam valve open at 75% capacity.

The temperature of the starch solution was then decreased to 55°C. 0.05% glucoamylase (AMG 200 L, commercially available from Novo Nordisk) based on the weight of the starch was added and the reaction was allowed to proceed at 55°C with constant mixing for approximately 2.5 hours until a dextrose equivalent of 36 and a viscosity of 17 sec at 25% solids and 22°C using the funnel method. The enzyme was then deactivated by heating the dispersion to 90°C and maintaining the elevated temperature for 30 minutes. The dispersion was then cooled to room temperature and spray dried using an inlet temperature of 200°C, an outlet temperature of 100°C, and a feed rate of 65 ml/min.

Example 3 - Encapsulation of Orange Oil with 40% Load

240 grams of the modified starch prepared in Example 2 was dispersed in 600 ml water in a high dispersion mill. The temperature was raised to 60°C until the starch dissolution appeared complete and then was lowered to 40°C. 160g of a single pressed orange oil commercially available from Givaudan-Roure was added and emulsified at high speed for approximately three minutes. The viscosity of the emulsion as determined using a Brookfield Viscometer Model 1+ using a small sample adaptor with spindle #18 is 85 mPas (cps) at 40°C. The emulsion was sprayed dried to a powder.

The resultant encapsulated orange oil retained 38% oil based on the weight of the product, a 95% encapsulation of the oil used in the system; the surface oil (extractable oil) was 0.3%; the oxidation was found to be at acceptable levels after aging, 0.8% Carvone; and the moisture of the product was 1.9% as determined by the Karl-Fischer Method.

Example 4 - Preparation of an Orange Drink Mix

| Ingredient | Amount (grams) |
|---|---|
| Example 3 encapsulated oil | 2.18 |
| Sugar | 95.58 |
| Citric Acid | 1.74 |
| FD&C Yellow #15 | 0.03 |
| FD&C Yellow #6 | 0.03 |
| Benzoic Acid | 0.44 |

The ingredients were dry blended to prepare a powdered orange drink mix. 11.51 grams of the mix were reconstituted with 88.5 ml water to produce a clean-tasting orange drink which was free from oxidized flavors.

Example 5 - Encapsulation of Vitamin E

a. 165 grams of the starch of example 2 were dispersed in 670 grams water in a high dispersion mill. The temperature was raised to 60°C until the starch dissolution appeared complete and then was lowered to 40°C. 165 g of Vitamin E was added and emulsified at high speed for approximately three minutes. The emulsion was spray dried to a powder which contained 50% of 1000 IU Vitamin E.
b. Example 5a was repeated using a zero bloom fish gelatin.
c. Example 5a was repeated using CAPSUL® starch, an encapsulating starch commercially available from National Starch and Chemical Company in Bridgewater, New Jersey.

Example 6 - Use of the Modified Starch in a Vitamin E Tablet

The encapsulated vitamin Es of Example 3 were made into compressed tablets using the following formulation.

| Ingredient | Amount (mg) |
|---|---|
| Encapsulated Vitamin E | 4450.0 |
| Magnesium Stearate[1] | 25.0 |
| Amorphous fumed silica[2] | 25.0 |

[1] Magnesium stearate is commercially available from Witco.

[2] Amorphous fumed silica is commercially available under the tradename Cab-O-Sil M5 P from Cabot.

(continued)

| Ingredient | Amount (mg) |
|---|---|
| Microcrystalline cellulose[3] | 500.0 |

[3] Microcrystalline cellulose is commercially available under the tradename Avicel PH102 from FMC.

The ingredients were dry blended, 500 mg portions were weighed out and loaded into the press to form tablets using a Riva Piccola 10-station lab scale press at 272, 680, and 2676 kg face (600, 1500, and 5900 pounds force). The hardness of these tablets were tested using a Pharmatron Model 6d Tablet Tester and disintegration was tested using an Erweka ZT71 Disintegration Tester.

 a. The encapsulated Vitamin E of example 5a was used. The results are listed below in Table I.
 b. The encapsulated Vitamin E of example 5b was used. The results are listed below in Table I.
 c. The encapsulated Vitamin E of example 5c was used. The results are listed below in Table I.

Table I

| Example | Compression Force kg (lbs) | Hardness (kP) | Disintegration (hh:mm:ss) |
|---|---|---|---|
| 6a | 224 (494) | 3.3 | 0:23:01 |
| | 832 (1835) | 3.7 | 0:16:43 |
| | 2026 (4466) | 3.6 | 0:16:47 |
| 6b | 351 (773) | 4.7 | 0:25:59 |
| | 803 (1770) | 4.6 | 0:24:25 |
| | 2004 (4417) | 5.8 | 0:22:01 |
| 6c | 212 (467) | 1.5 | 0:10:38 |
| | 398 (877) | 1.3 | 0:16:04 |
| | 1885 (4156) | 1.1 | 0:09:34 |

As can be seen from Table I, the encapsulating starches of the present invention provide good compressibility and suitable tablet hardness. In many cases, the present invention was at least comparable to the standard gelatin. Further, different hardnesses may be achieved by varying the formulation and the compression force.

Example 7 - Use of the Modified Starch in a Vitamin E Tablet

Example 6 was repeated using the following formulation:

| Ingredient | Amount (mg) |
|---|---|
| Encapsulated Vitamin E | 4700.0 |
| Magnesium Stearate[1] | 25.0 |
| Amorphous fumed silica[2] | 25.0 |
| Microcrystalline cellulose[3] | 250.0 |

 a. The encapsulated Vitamin E of example 3a was used. The results are listed below in Table II.
 b. The encapsulated Vitamin E of example 3b was used. The results are listed below in Table II.
 c. The encapsulated Vitamin E of example 3c was used. The results are listed below in Table II.

Table II

| Example | Compression Force kg (lbs) | Hardness (kP) | Disintegration (hh:mm:ss) |
|---|---|---|---|
| 7a | 257 (566) | 1.4 | 0:11:29 |
| | 629 (1386) | 1.68 | 0:13:33 |
| | 2414 (5322) | 1.28 | 0:11:46 |
| 7b | 256 (565) | 1.34 | 0:23:21 |
| | 555 (1223) | 1.92 | 0:24:39 |
| | 2514 (5543) | 2.16 | 0:18:46 |
| 7c | 229 (504) | 0.32 | 0:11:53 |
| | 738 (1627) | 0.46 | 0:13:55 |
| | 2416 (5326) | 0.18 | 0:15:28 |

As can be seen from Table II, the encapsulating starches of the present invention provide good compressibility and suitable tablet hardness. In many cases, the present invention was at least comparable to the standard gelatin. Further, different hardnesses may be achieved by varying the formulation and the compression force.

Example 8- Analysis of Encapsulation

A variety of glucoamylase degraded OSA starches were prepared using the method of Example 2 with the exception that hydrolysis was allowed to proceed to different degrees. The modified starches were used to encapsulate orange oil as in Example 3. The results of the encapsulation as reported below.

| Enzyme | DE | Viscosity at 22°C (sec) | | %Retention | | %Surface Oil | %Carvone |
|---|---|---|---|---|---|---|---|
| | | 19% solids | 25% solids | Time=0 | Time = 2 weeks at 50°C | | |
| Glucoamylase | 19.7 | --- | --- | 84.21 | 73.68 | 3.87 | 2.10 |
| Glucoamylase | 30.3 | 10.7 | 19.8 | 86.61 | 78.95 | 1.31 | 1.12 |
| Glucoamylase | 37.7 | 9.7 | 15.3 | 98.31 | 95.51 | 0.08 | 0.36 |
| Glucoamylase | 48.3 | 7.7 | 9.5 | 91.62 | 89.47 | 0.19 | 0.58 |
| Glucoamylase | 48.3 | 7.7 | 9.5 | 91.62 | 89.24 | 0.05 | .029 |
| Glucoamylase | 30 | --- | --- | 98.20 | 81.24 | 1.94 | 1.06 |
| Glucoamylase | 45 | --- | --- | 91.60 | 91.20 | 0.05 | 0.52 |

Example 9- Comparison with other encapsulating starches

Starch example 9a is a 50:50 blend of corn syrup solids and cold water soluble, acid degraded, OSA starch in which the starch is prepared by acid hydrolyzing the OSA starch of Example 1 to a water fluidity of about 65 and then spray drying.

Example 9b is the OSA starch of example 1.

The modified starches were used to encapsulate orange oil as in Example 3. The results of the encapsulation as reported below.

| Example | DE | %Retention | | %Surface Oil | %Carvone |
|---|---|---|---|---|---|
| | | Time=0 | Time = 2 weeks at 50°C | | |
| 9a | 23 | 84.20 | 74.40 | 4.54 | 1.03 |
| 9b | 0 | 56.0 | --- | 12.0 | --- |

No stability test was run on example 9b due to the poor retention. A comparison of the above results with those of example 8 shows the superior retention, and decreased surface oil and oxidation of the present starches.

Example 10 - Preparation of an Antiperspirant

| Ingredient | Amount (grams) |
|---|---|
| Encapsulated fragrance | 1.00 |
| Dow Corning Fluid 344 | 49.0 |
| Cyclochem EDGS | 1.00 |
| Arlacel 165 | 1.00 |
| Promyristyl PM3 | 5.00 |
| Crodacol S-95NF | 17.00 |
| Rezal 36 GP Suf | 20.00 |
| DRY-FLO® PC Starch | 6.00 |

The encapsulated fragrance is prepared by the methodology of Example 3, substituting the fragrance for the orange oil.

Dow Corning Fluid 344 is cyclomethicone commercially available from Dow Corning. Cyclochem EDGS is glycol distearate commercially available from Alcolac.

Arlacel 165 is glyceryl stearate and PEG 100 stearate commercially available from ICI.

Promyristyl PM3 is PPG-3 myristyl ether commercially available from Croda.

Crodacol S-95NF is stearyl alcohol commercially available from Croda.

Rezal 36 GP Suf is aluminum zirconium tetrachlorohydrex glycine commercially available from Reheis.

DRY-FLO® PC starch, a modified food starch used as a dusting and lubricating agent, is commercially available from National Starch and Chemical Company.

The Dow Corning Fluid 344, Cyclochem EDGS, Arlacel 165, and Promyristyl PM3 are mixed and heated to 65°C. The Crodacol S-95NF is mixed in thoroughly. The Rezal 36 GP Suf is added and mixed for ten minutes. The DRY-FLO® starch is added and mixed thoroughly. Then the encapsulated fragrance is added and mixed thoroughly. The mixture is cooled to 50°C, poured into molds, and cooled to room temperature.

Example 11 - Preparation of a Detergent

| Ingredient | Amount (g) |
|---|---|
| Sodium lauryl sulfate | 15 |
| Sodium lauryl ether sulphate | 10 |
| Magnesium aluminum silicate | 30 |
| Sodium Carbonate | 19.5 |
| Sodium disilicate | 2 |
| Sodium Perborate | 15 |
| Sodium polycarboxylate (acrylic/maleic) | 3 |
| Polyester terephthalate | 2 |
| Protease enzyme | 0.5 |
| Encapsulated fragrance | 0.75 |
| Optical brightener | 0.2 |

(continued)

| Ingredient | Amount (g) |
|---|---|
| Sodium phosphonate | 1.55 |
| TAED bleach activator | 0.5 |

The fragrance is encapsulated using the starch of Example 2 and the method of Example 3 in which the fragrance is substituted for the orange oil. The ingredients are blended together.

Example 12 - Preparation of a Phosphate-Free Detergent

| Ingredient | Amount (g) |
|---|---|
| Magnesium Aluminum Silicate | 30 |
| Sodium Percarbonate | 15 |
| Sodium Alkyl Sulphate | 15 |
| Sodium Alcohol Ethoxylate | 10 |
| Dimethylamine Oxide | 6.75 |
| Sodium Carbonate | 15 |
| Sodium Sulphate | 7 |
| Encapsulated Fragrance | 1.25 |

The fragrance is encapsulated using the starch of Example 2 and the method of Example 3 in which the fragrance is substituted for the orange oil. The ingredients are blended together.

Example 13 - Preparation of an Orange Oil Emulsion

| Ingredient | Amount (g) |
|---|---|
| Orange oil (one fold) | 5.8 |
| Orange oil (five fold) | 1.4 |
| Ester gum | 4.8 |
| Starch | 12 |
| Sodium benzoate | 0.15 |
| Citric acid | 0.30 |
| Water | 75.55 |

The starch used was that of example 2 except that the hydrolysis was allowed to proceed to a DE of 26 and a viscosity of 28 sec at 25% solids and 22°C using the funnel method.

The ester gum was thoroughly dissolved in the mixture of the orange oils at room temperature using constant agitation. The sodium benzoate was then dissolved in the water after which the citric acid was dissolved in the solution. The starch was then dispersed in the solution using moderate agitation. The orange oil/ester gum was then added slowly with moderate agitation and the mixture was homogenized at 210 kg/cm$^2$ (3000 psi) and then at 360 kg/cm$^2$ (5000 psi). The resultant emulsion has a viscosity of 75 cps using a Brookfied Viscometer with a #2 spindle at 60 rpm, a pH of 3.46, a median particle size of 0.265 microns and a mean particle size of 0.276.

Example 14 - Preparation of a Beverage Using the Emulsion

| Ingredient | Amount (g) |
|---|---|
| Sugar | 11 |
| Emulsion of Example 13 | 0.015 |
| Sodium benzoate | 0.05 |
| Citric Acid | 0.2 |
| Yellow #5 | 0.004 |
| Yellow #6 | 0.004 |
| Water | 88.73 |

The sodium benzoate was dissolved in water. The citric acid, color, and sugar were then added. The emulsified orange oil was then added. There was no ringing upon storage of the beverage.

**Claims**

1. A modified starch comprising a starch derivative containing a hydrophobic group or both a hydrophobic and a hydrophilic group which has been degraded by glucoamylase.

2. The starch of claim 1, wherein the starch is degraded to a dextrose equivalent of from 20 and up to 80

3. The starch of claim 2, wherein the starch is degraded to a dextrose equivalent of from 30 to 50.

4. The starch of any one of claims 1-3, wherein the starch is selected from the group consisting of corn, waxy maize, tapioca, potato, and rice.

5. The starch of any one of claims 1-4, wherein the starch has a viscosity of less than 30 seconds as measured by the funnel method.

6. The starch of claim 5, wherein the starch has a viscosity of from 8 to 25 seconds.

7. The starch of claim 6, wherein the starch has a viscosity of from 8 to 15 seconds.

8. The starch of any one of claims 1-7, wherein the starch derivative is gelatinized and the hydrophobic group comprises an alkyl or an alkenyl group which contains at least five carbon atoms or an aralkyl or aralkenyl group which contains at least six carbon atoms.

9. The starch of claim 1, wherein the starch is gelatinized and has been derivatized by treatment with at least 0.1% of octenyl succinic acid anhydride on a starch dry weight basis.

10. The starch of any one of claims 1-9, wherein the starch has encapsulation properties **characterized by** at least 40% load capacity, low surface oil, and improved oxidation resistance.

11. A method for preparing a modified starch comprising:

    a. derivatizing a starch such that it contains a hydrophobic group or a hydrophobic and a hydrophilic group; and
    b. degrading the starch using glucoamylase.

12. The method of claim 11, wherein the starch is degraded to a dextrose equivalent of from 20 and up to 80.

13. The method of claim 12, wherein the starch is degraded to a dextrose equivalent of from 30 to 50.

14. The method of any one of claims 11-13, wherein the starch is selected from the group consisting of corn, waxy maize, tapioca, potato, and rice.

15. The method of any one of claims 11-14, further comprising the step of gelatinizing the starch prior to degradation.

16. The method of any one of claims 11-15, wherein the hydrophobic group comprises an alkyl or an alkenyl group which contains at least five carbon atoms or an aralkyl or aralkenyl group which contains at least six carbon atoms.

17. The method of claim 16, wherein the derivatization is carried out using at least 0.1% of octenyl succinic acid anhydride on a starch dry weight basis.

18. The method of any one of claims 11-17, wherein the modified starch has encapsulating properties **characterized by** at least 40% load capacity, low surface oil, and improved oxidation resistance.

19. The method of any one of claims 11-18, wherein the modified starch has emulsion properties **characterized by** improved stability and resistance to oiling and gelling during storage.

20. An encapsulating agent comprising the starch of any one of claims 1-10.

21. An emulsifying agent comprising the starch of any one of claims 1-10.

22. A method of encapsulating an active agent comprising the starch of any one of claims 1-10, the method comprising:

   a) forming a solution of the encapsulating agent; and
   b) emulsifying the active agent in the solution.

23. The method of claim 22, further comprising drying the emulsion to remove the water therefrom.

24. The method of claim 23, wherein the drying step is accomplished by spray drying.

25. A method of emulsifying an active agent comprising the starch of any one of claims 1-10, the method comprising:

   a) forming a solution of the encapsulating agent; and
   b) emulsifying the active agent in the solution.

26. A composition comprising the encapsulating agent of claim 20.

27. A composition comprising the emulsifying agent of claim 21.

28. An emulsion comprising the modified starch of any one of claims 1-10 10.

29. A tablet comprising

   a) an encapsulating agent comprising the starch of any one of claims 1-10; and
   b) an active agent.

30. The tablet of claim 29, wherein the active agent is selected from the group consisting of a vitamin, a pharmaceutical, a pesticide, an oil, a protein, a fat, a flavor, a color, a catalyst, and a fragrance.

31. The tablet of claim 29 or 30, wherein the active agent is selected from the group consisting of a botanical oil, an essential oil; Vitamin A, Vitamin D, Vitamin E, Vitamin K; a peptide, an amino acid, an enzyme; and an oil soluble pharmaceutical, oil soluble pesticide.

32. The tablet of claim 31, wherein the active agent is Vitamin E.

33. The tablet of any one of claims 29-32, wherein the tablet has a hardness of at least 3 kilopascals.

34. The tablet of any one of claims 29-32, wherein the tablet has a hardness of at about 4 kilopascals.

**EP 0 913 406 B1**

**Patentansprüche**

1. Modifizierte Stärke, die ein Stärkederivat, das eine hydrophobe Gruppe oder sowohl eine hydrophobe Gruppe als auch eine hydrophile Gruppe enthält, umfaßt, und die durch Glucoamylase abgebaut wurde.

2. Stärke nach Anspruch 1, wobei die Stärke zu einem Dextroseäquivalent von 20 bis 80 abgebaut wurde.

3. Stärke nach Anspruch 2, wobei die Stärke zu einem Dextroseäquivalent von 30 bis 50 abgebaut wurde.

4. Stärke nach einem der Ansprüche 1 bis 3, wobei die Stärke aus der Gruppe bestehend aus Mais, Wachsmais, Tapoika, Kartoffeln und Reis ausgewählt ist.

5. Stärke nach einem der Ansprüche 1 bis 4, wobei die Stärke eine Viskosität von weniger als 30 Sekunden, gemessen durch das Tunnelverfahren, hat.

6. Stärke nach Anspruch 5, wobei die Stärke eine Viskosität von 8 bis 25 Sekunden hat.

7. Stärke nach Anspruch 6, wobei die Stärke eine Viskosität von 8 bis 15 Sekunden hat.

8. Stärke nach einem der Ansprüche 1 bis 7, wobei das Stärkederivat geliert ist und die hydrophobe Gruppe eine Alkyl- oder eine Alkenyl-Gruppe, die mindestens 5 Kohlenstoffatome enthält, oder eine Aralkyl- oder Aralkenyl-Gruppe, die mindestens 6 Kohlenstoffatome enthält, umfaßt.

9. Stärke nach Anspruch 1, wobei die Stärke geliert ist und durch Behandlung mit mindestens 0,1 % Octenylbernsteinsäureanhydrid, bezogen auf das Stärke-Trockengewicht, derivatisiert wurde.

10. Stärke nach einem der Ansprüche 1 bis 9, wobei die Stärke Einkapselungseigenschaften, **gekennzeichnet durch** mindestens 40 % Beladungskapazität, geringes Oberflächenöl und verbesserte Oxidationsbeständigkeit, hat.

11. Verfahren zur Herstellung einer modifizierten Stärke, umfassend:

    a) Derivatisieren einer Stärke derart, daß sie eine hydrophobe Gruppe oder eine hydrophobe Gruppe und eine hydrophile Gruppe enthält, und

    b) Abbauen der Stärke unter Verwendung von Glucoamylase.

12. Verfahren nach Anspruch 11, wobei die Stärke zu einem Dextroseäquivalent von 20 bis 80 abgebaut wird.

13. Verfahren nach Anspruch 12, wobei die Stärke zu einem Dextroseäquivalent von 30 bis 50 abgebaut wird.

14. Verfahren nach einem der Ansprüche 11 bis 13, wobei die Stärke aus der Gruppe bestehend aus Mais, Wachsmais, Tapioka, Kartoffeln und Reis ausgewählt wird.

15. Verfahren nach einem der Ansprüche 11 bis 14, das außerdem die Stufe der Gelierung der Stärke vor einem Abbau umfaßt.

16. Verfahren nach einem der Ansprüche 11 bis 15, wobei die hydrophobe Gruppe eine Alkyl- oder Alkenyl-Gruppe, die mindestens 5 Kohlenstoffatome enthält, oder eine Aralkyl- oder Aralkenyl-Gruppe, die mindestens 6 Kohlenstoffatome enthält, umfaßt.

17. Verfahren nach Anspruch 16, wobei die Derivatisierung unter Verwendung von mindestens 0,1 % Octenylbernsteinsäureanhydrid, bezogen auf das Stärke-Trockengewicht, durchgeführt wird.

18. Verfahren nach einem der Ansprüche 11 bis 17, wobei die modifizierte Stärke Einkapselungseigenschaften, **gekennzeichnet durch** mindestens 40 % Beladungskapazität, geringes Oberflächenöl und verbesserte Oxidationsbeständigkeit, hat.

19. Verfahren nach einem der Ansprüche 11 bis 18, wobei die modifizierte Stärke Emulsionseigenschaften, die durch verbesserte Stabilität und verbesserte Beständigkeit gegen Öle und Gelieren während der Lagerung **gekennzeichnet** sind, hat.

**20.** Einkapselungsmittel, das die Stärke nach einem der Ansprüche 1 bis 10 umfaßt.

**21.** Emulgiermittel, das die Stärke nach einem der Ansprüche 1 bis 10 umfaßt.

**22.** Verfahren zur Einkapselung eines aktiven Agenzes, das die Stärke nach einem der Ansprüche 1 bis 10 umfaßt, wobei das Verfahren umfaßt:

a) Bildung einer Lösung des Einkapselungsmittels und

b) Emulgieren des aktiven Agenzes in der Losung.

**23.** Verfahren nach Anspruch 22, das außerdem Trocknen der Emulsion unter Entfernung des Wassers daraus umfaßt.

**24.** Verfahren nach Anspruch 23, wobei die Trocknungsstufe durch Sprühtrocknung durchgeführt wird.

**25.** Verfahren zum Emulgieren eines aktiven Agenzes, das die Stärke nach einem der Ansprüche 1 bis 10 umfaßt, wobei das Verfahren umfaßt:

a) Bildung einer Lösung des Einkapselungsmittels und

b) Emulgieren des aktiven Agenzes in der Lösung.

**26.** Zusammensetzung, die das Einkapselungsmittel nach Anspruch 20 umfaßt.

**27.** Zusammensetzung, die das Emulgiermittel nach Anspruch 21 umfaßt.

**28.** Emulsion, die die modifizierte Starke nach einem der Ansprüche 1 bis 10 umfaßt.

**29.** Tablette, umfassend:

a) ein Einkapselungsmittel, das die Stärke nach einem der Ansprüche 1 bis 10 umfaßt, und

b) ein aktives Agens.

**30.** Tablette nach Anspruch 29, wobei das aktive Agens aus der Gruppe bestehend aus einem Vitamin, einem Arzneimittel, einem Pestizid, einem Öl, einem Protein, einem Fett, einem Aromastoff, einem Farbstoff, einem Katalysator und einem Duftmittel ausgewählt ist.

**31.** Tablette nach Anspruch 29 oder 30, wobei das aktive Agens aus der Gruppe bestehend aus einem botanischem Öl, einem essentiellen Öl; Vitamin A, Vitamin D, Vitamin E, Vitamin K, einem Peptid, einer Aminosäure, einem Enzym und einem öllöslichen Arzneimittel, öllöslichem Pestizid ausgewählt ist.

**32.** Tablette nach Anspruch 31, wobei das aktive Agens Vitamin E ist.

**33.** Tablette nach einem der Ansprüche 29 bis 32, wobei die Tablette eine Härte von mindestens 3 Kilopascal hat.

**34.** Tablette nach einem der Ansprüche 29 bis 32, wobei die Tablette eine Härte von etwa 4 Kilopascal hat.

**Revendications**

**1.** Amidon modifié comprenant un dérivé d'amidon contenant un groupe hydrophobe ou bien à la fois un groupe hydrophobe et un groupe hydrophile, qui a été dégradé par la gluco-amylase.

**2.** Amidon suivant la revendication 1, dans lequel l'amidon est dégradé à un équivalent en dextrose de 20 jusqu'à 80.

**3.** Amidon suivant la revendication 2, dans lequel l'amidon est dégradé à un équivalent en dextrose de 30 à 50.

**4.** Amidon suivant l'une quelconque des revendications 1 à 3, dans lequel l'amidon est choisi dans le groupe consistant en les amidons de mais, de mais cireux, de manioc, de pomme de terre et de riz.

**5.** Amidon suivant l'une quelconque des revendications 1 à 4, dans lequel l'amidon a une viscosité inférieure à 30 secondes, mesurée par la méthode à l'entonnoir.

**6.** Amidon suivant la revendication 5, dans lequel l'amidon a une viscosité de 8 à 25 secondes.

**7.** Amidon suivant la revendication 6, dans lequel l'amidon a une viscosité de 8 à 15 secondes.

**8.** Amidon suivant l'une quelconque des revendications 1 à 7, dans lequel le dérivé d'amidon est gélatinisé et le groupe hydrophobe comprend un groupe alkyle ou alcényle qui contient au moins 5 atomes de carbone ou un groupe aralkyle ou aralcényle qui contient au moins 6 atomes de carbone.

**9.** Amidon suivant la revendication 1, dans lequel l'amidon est gélatinisé et a été transformé en dérivé par traitement avec au moins 0,1% d'anhydride d'acide octénylsuccinique sur la base du poids sec de l'amidon.

**10.** Amidon suivant l'une quelconque des revendications 1 à 9, dans lequel l'amidon a des propriétés d'encapsulation **caractérisées par** une capacité de charge d'au moins 40%, une faible émission d'huile en surface et une résistance à l'oxydation améliorée.

**11.** Procédé pour la préparation d'un amidon modifié, comprenant les étapes consistant :

a) à transformer en dérivé un amidon de telle sorte qu'il contienne un groupe hydrophobe ou bien un groupe hydrophobe et un groupe hydrophile ; et
b) à dégrader l'amidon en utilisant de la gluco-amylase

**12.** Procédé suivant la revendication 11, dans lequel l'amidon est dégradé à un équivalent en dextrose de 20 jusqu'à 80.

**13.** Procédé suivant la revendication 12, dans lequel l'amidon est dégradé à un équivalent en dextrose de 30 à 50.

**14.** Procédé suivant l'une quelconque des revendications 11 à 13, dans lequel l'amidon est choisi dans le groupe consistant en les amidons de maïs, de maïs cireux, de manioc, de pomme de terre et de riz.

**15.** Procédé suivant l'une quelconque des revendications 11 à 14, comprenant en outre l'étape consistant à gélatiniser l'amidon avant dégradation.

**16.** Procédé suivant l'une quelconque des revendications 11 à 15, dans lequel le groupe hydrophobe comprend un groupe alkyle ou alcényle qui contient au moins 5 atomes de carbone ou un groupe aralkyle ou aralcényle qui contient au moins 6 atomes de carbone.

**17.** Procédé suivant la revendication 16, dans lequel la transformation en dérivé est effectuée en utilisant au moins 0,1% d'anhydride d'acide octénylsuccinique sur la base du poids sec de l'amidon.

**18.** Procédé suivant l'une quelconque des revendications 11 à 17, dans lequel l'amidon modifié a des propriétés d'encapsulation **caractérisées par** une capacité de charge d'au moins 40%, une faible émission d'huile en surface et une résistance à l'oxydation améliorée.

**19.** Procédé suivant l'une quelconque des revendications 11 à 18, dans lequel l'amidon modifié a des propriétés d'émulsionnement **caractérisées par** une stabilité améliorée et une résistance améliorée à la formation d'huile et à la gélification au cours du stockage.

**20.** Agent d'encapsulation comprenant l'amidon suivant l'une quelconque des revendications 1 à 10.

**21.** Agent émulsionnant comprenant l'amidon suivant l'une quelconque des revendications 1 à 10.

**22.** Procédé d'encapsulation d'un agent actif comprenant l'amidon suivant l'une quelconque des revendications 1 à 10, procédé comprenant les étapes consistant :

    a) à former une solution de l'agent d'encapsulation
    b) à émulsionner l'agent actif dans la solution.

**23.** Procédé suivant la revendication 22, comprenant en outre le séchage de l'émulsion pour en éliminer l'eau.

**24.** Procédé suivant la revendication 23, dans lequel l'étape de séchage est mise en oeuvre par séchage par pulvérisation.

**25.** Procédé pour émulsionner un agent actif comprenant l'amidon suivant l'une quelconque des revendications 1 à 10, procédé comprenant les étapes consistant :

    a) à former une solution de l'agent d'encapsulation
    b) à émulsionner l'agent actif dans la solution.

**26.** Composition comprenant l'agent d'encapsulation suivant la revendication 20.

**27.** Composition comprenant l'agent d'émulsionnement suivant la revendication 21.

**28.** Emulsion comprenant l'amidon modifié suivant l'une quelconque des revendications 1 à 10.

**29.** Comprimé comprenant

    a) un agent d'encapsulation comprenant l'amidon suivant l'une quelconque des revendications 1 à 10 ;
    b) un agent actif.

**30.** Comprimé suivant la revendication 29, dans lequel l'agent actif est choisi dans le groupe consistant en une vitamine, un agent pharmaceutique, un pesticide, une huile, une protéine, une matière grasse, un arôme, un colorant, un catalyseur et un parfum.

**31.** Comprimé suivant la revendication 29 ou 30, dans lequel l'agent actif est choisi dans le groupe consistant en une huile végétale, une huile essentielle : la vitamine A, la vitamine D, la vitamine E, la vitamine K ; un peptide, un amino-acide, une enzyme ; un agent pharmaceutique soluble dans l'huile et un pesticide soluble dans l'huile.

**32.** Comprimé suivant la revendication 31, dans lequel l'agent actif est la vitamine E.

**33.** Comprimé suivant l'une quelconque des revendications 29 à 32, qui a une dureté d'au moins 3 kilopascals.

**34.** Comprimé suivant l'une quelconque des revendications 29 à 32, qui a une dureté d'environ 4 kilopascals.